# EUROPEAN PATENT APPLICATION

(11) **EP 1 045 026 A1**
(43) Date of publication of application: **18.10.2000**
(21) Application number: 99106270.4
(22) Date of filing: 16.04.1999
(51) Int. Cl.: C12N 15/00, C12N 5/00, A61K 49/00, A01K 67/027

(54) **A transgenic coagulation factor XIII defective animal and its use for testing wound healing and bleeding**

(71) Applicant: Aventis Behring Gesellschaft mit beschränkter Haftung, 35002 Marburg (DE)
(72) Inventor: Dickneite, Gerhard, Dr., 35043 Marburg (DE); Metzner, Hubert, Dr., 35041 Marburg (DE); Zettlmeissl, Gerd, Dr., 35083 Wetter (DE); Grundmann, Ulrich, Dr., 35094 Lahntal (DE); Lathe, Richard, Prof., Centre for Genome Research, Edinburgh EH9 3JQ (GB); Smith, Austin, Dr., Centre for Genome Research, Edinburgh EH9 3JQ (GB); Li, Meng, Dr., Centre for Genome Research, Edinburgh EH9 3JQ (GB)

(57) **Abstract**

The invention provides a transgenic non-human mammalian animal which is heterozygous or homozygous for an at least partially defective coagulation factor XIII gene.

## Description

This invention relates to a transgenic non-human mammalian animal which is heterozygous or homozygous for an at least partially defective coagulation factor XIII gene.

Factor XIII is an enzyme of the coagulation cascade which cross-links fibrin and thus promotes the stability of the hemostatic plug.

The coagulation is a cascade system of proteolytic enzymes with subsequent activation by limited proteolysis. After wounding the intrinsic and the extrinsic pathways of coagulation are activated. The intrinsic activation includes coagulation factors XII, XI and IX which are proteolytic enzymes (proteases) and an enzyme cofactor (factor VIII). The extrinsic system includes tissue factor and factor VII. Both activation pathways result in the common pathway containing the protease factor Xa and its cofactor (factor Va) which convert the inactive prothrombin (factor II) to the enymatically active thrombin (IIa, n.b.: activated factors are marked with an "a" after the roman number, the roman number alone marks the inactive enzyme). Thrombin is considered to be the central protease of the coagulation system. It cleaves fibrinogen (factor I) resulting in fibrin which is the substrate for the wound closure. Together with blood platelets fibrin forms the hemostatic plug. Thrombin moreover activates the clotting factor XIII by limited proteolysis. In contrast to the other proteases of the coagulation cascade, factor XIII is a transglutaminase. Factor XIII cross-links the fibrin molecule via lysine and glutamine residues, rendering soluble fibrin into insoluble fibrin.

FXIII as found in plasma is composed to two nonidentical subunits, the a-chain and b-chain, with molecular weights of approximately 83.000 and 76.500 Da respectively (McDonagh J., in : Hemostasis and Thrombosis, Colman RW, Hirsh J., March V.J. and Salzmann E.W. (eds), Lippincott, Philadelphia, 1994).

The tetrameric complex in plasma has the composition a₂b₂ with a Mr of 320 000 Da. The concentration of the tetramer in plasma is about 0.07 µmol/l. During the activation step, thrombin cleaves an Arg-Gly bond at the positions 37 and 38 of the a-chain releasing a Mr 4500 activation peptide from the amino terminus. The b-chain dimer dissociates from the complex to release the active a-chain dimer (a₂*, FXIIIa). FXIIIa is a glutamine-lysine transferase. The reaction, catalyzed is the formation of an isopeptide bond between the γ-carbonyl group of glutamine and the ε-amino group of lysine between fibrin molecules, stabilizes the fibrin network. Four to six lysyl-glutamyl crosslinks are formed per mole of fibrin.

The lack of factor XIII in patients might lead to hemorrhagic diathesis and impairment of wound healing (Duckert F., Ann NY Acad Sci, vol. 202, 190-199, 1972). Patients with a congenital FXIII deficiency suffer from soft tissue and joint hemorrhage after trauma. The most serious form of bleeding is in the central nervous system, FXIII deficient patients have a high incidence of intracranial hemorrhage. Women with a FXIII deficiency often undergo abortion during pregnancy. FXIII stimulates the proliferation of fibroblasts, which is essential for wound healing. Thus, patients with FXIII deficiency were reported to have abnormal wound healing.

In this invention the generation of a transgenic coagulation factor XIII defective mouse with disturbance of the wound healing process and bleeding disorders is described. Furthermore, its use is described to develop wound healing accelerating drugs or drugs normalizing coagulation.

A transgenic animal which carries an at least partially defective coagulation factor XIII gene may be prepared by known gene manipulations, in which the factor XIII gene will be inactivated by an insertion, deletion, substitution or inversion or other suitable genetic manipulations. In the performance of the present invention a transgenic animal with a defective coagulation factor XIII gene has been prepared by a targeted disruption of the gene for the factor XIIIa subunit with deletion of the active site encoding the exon 7 sequence.

Thus, according to another aspect of the present invention there is provided a method of producing a transgenic non-human mammalian animal with an at least partially defective coagulation factor XIII gene comprising:
1. preparing a DNA construct encoding an at least partially defective coagulation factor XIII gene;
2. introducing such DNA construct into suitable carrier cells;
3. identifying cells in which the DNA construct has integrated by homologous recombination;
4. inserting those targeted cells into an embryo;
5. placing the embryo into a mother animal, and allowing the embryo to develop to full term;
6. deriving offsprings heterozygous for the disrupted factor Xllla gene, and
7. interbreeding heterozygous offsprings to obtain animals homozygous for the defective factor Xllla gene.

A transgenic mouse has been prepared as follows:

### Preparation of a transgenic mouse

Homologous recombination in embryonic stem (ES) cells was employed to generate mice deficient for factor XIII.

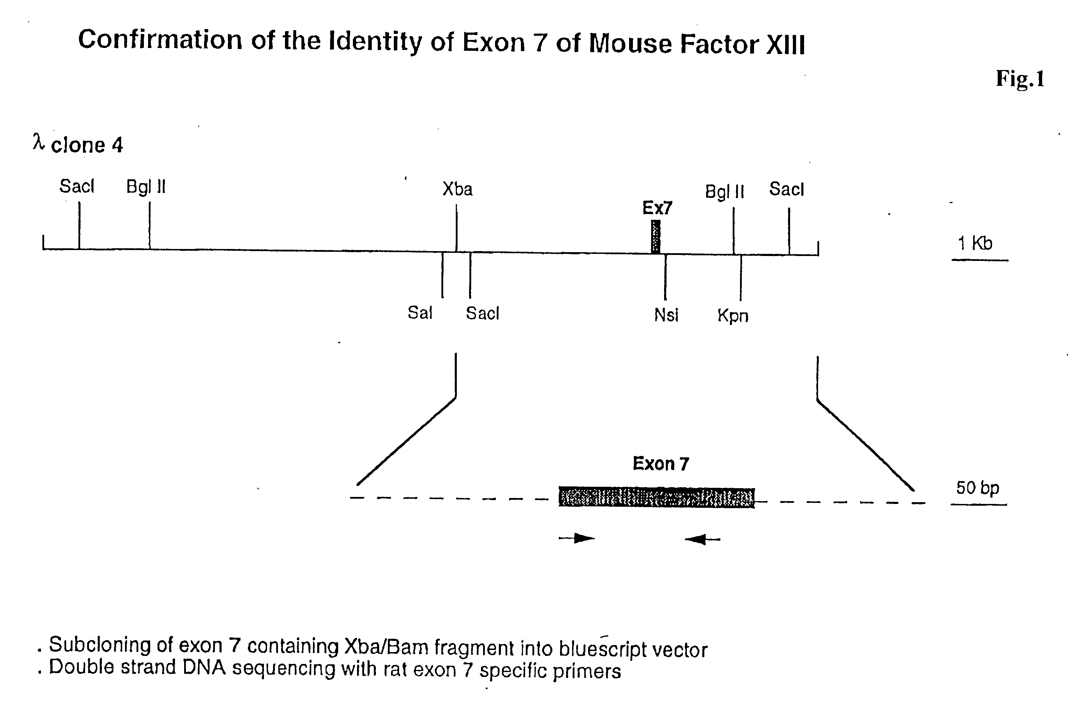

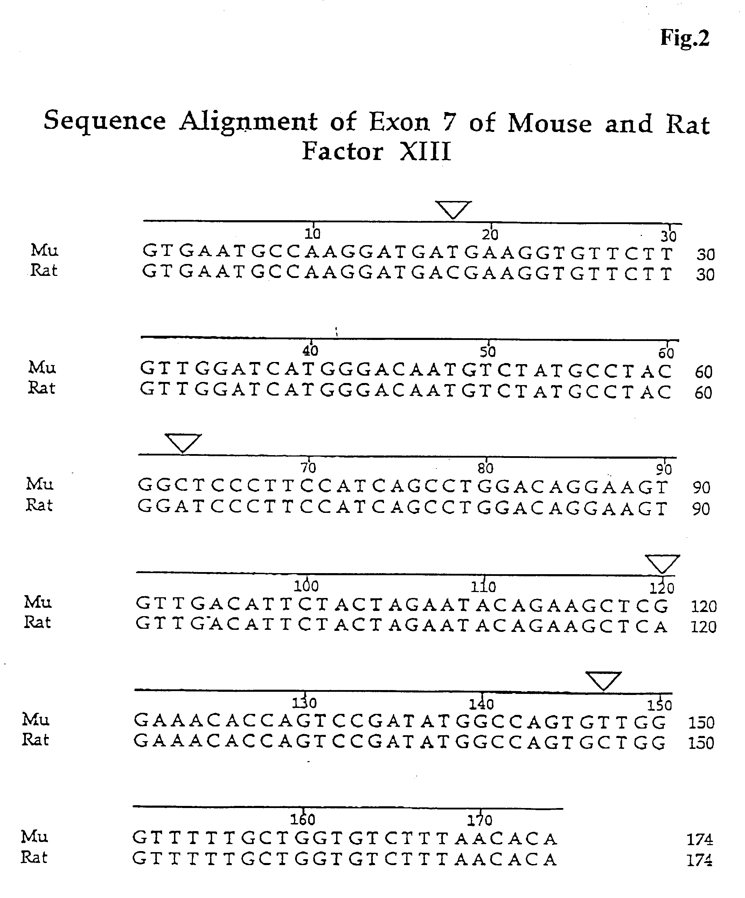

Mouse genomic sequences for the factor Xllla subunit were isolated from a strain 129 genomic library using rat cDNA sequence as probe. A probe specific for the active site encoding exon (exon 7) was generated by PCR amplification of a 110 bp cognate fragment of the rat cDNA. This probe was used to isolate a 14kb lambda clone (Figure 1). The presence of mouse factor XIII exon 7 sequence was confirmed by DNA sequencing which revealed 170/174 nucleotide identities with the rat exon 7 sequence (Figure 2 and Sequence Listing). The encoded amino acid sequence is completely identical to the rat protein sequence.

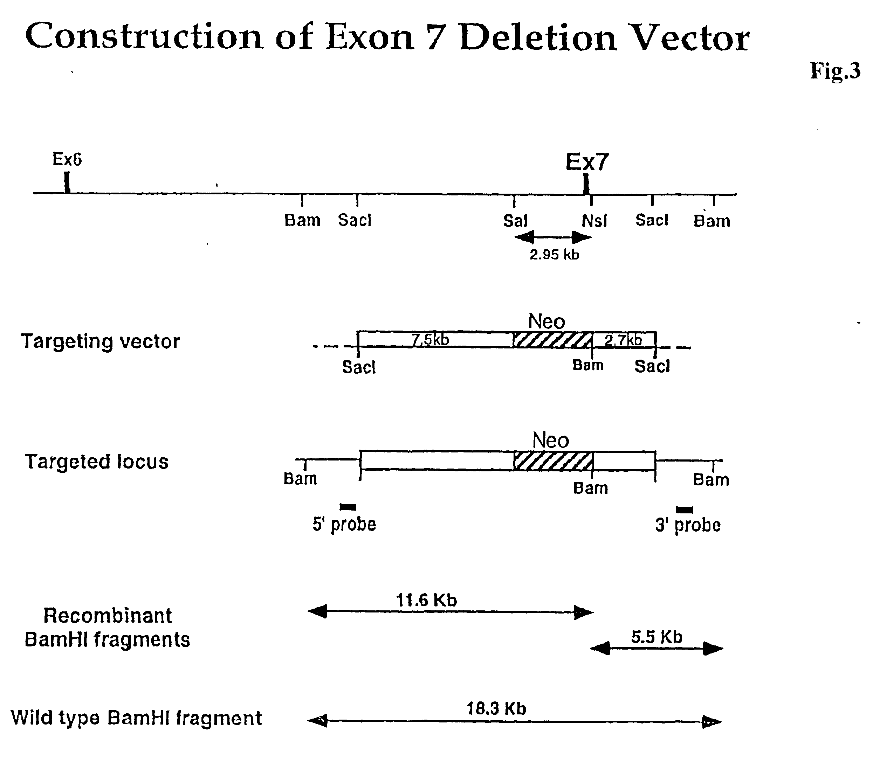

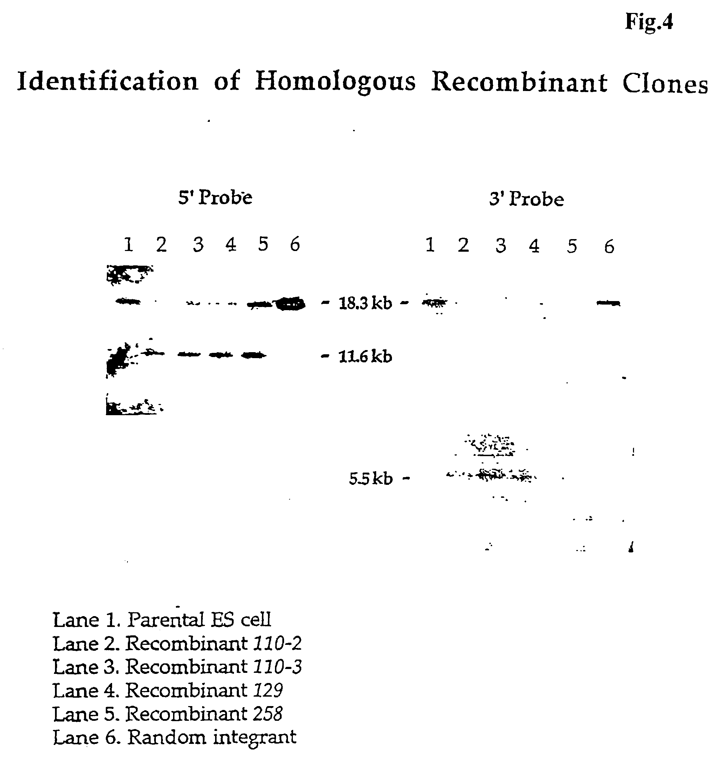

A replacement type targeting vector was constructed using standard recombinant DNA methods. An approximately 2.95kb region of genomic DNA encompassing exon 7 is deleted in the targeting construct and replaced by a b-actin promoter driven neomycin phosphotransferase selectable marker cassette (Figure 3). The targeting construct was digested with Sac 1 to excise the plasmid sequences, then introduced into E14TG2a ES cells by electroporation. Stable transfectants were isolated by selection in G418. Homologous recombinants were identified by Southern hybridisation of genomic DNA with probes external to both 5' and 3' homology regions (Figure 4). From two independent electroporations 4 out of 278 clones analysed gave the predicted hybridisation pattern for the homologous replacement event. Two of these clones (FXIII-110 and FXIII-129) were microinjected into C57BL/6 blastocyts to produce chimaeras. Chimaeras were initially test-crossed with outbred Swiss albino mice. Germline transmission was obtained from both clones.

Transmitting chimaeras were crossed with CBA/Ca mice to derive 129Ola x CBA offspring carrying the mutated gene for the Factor Xllla subunit. Following a backcross generation to CBA/Ca, heterozygous mice were intercrossed. Homozygous animals were identified by Southern hybridisation. The absence of exon 7 sequence in the homozygotes was confirmed using an exon 7 specific probe.

Said mouse has been characterized by its transglutaminase activity.

### Characterization of the factor XIII defective mouse by its transglutaminase activity

Venous blood was withdrawn and citrate plasma was obtained by centrifugation for further testing of transglutaminase activity. The assay used was the commercially available Berichrom F XIII kit, manufactured by Behring Diagnostics. As reference material human standard plasma has been used. Table 1 shows the results of the plasma transglutaminase determinations of normal mice with the same genetic background but without the defect in the FXIII gene, of hetereozygous FXIII defective mice and of homozgygous FXIII defective mice. The transglutaminase levels of the normal mice determined by Berichrom FXIII was 134%, for the heterozygous deficient animals the level determined was roughly half of that (70%). The transglutaminase levels of the homozygous FXIII defective mice was < 6% . This could be confirmed with another FXIII assay, a clot stability assay where the FXIII activity is determined more specifically by its cross-linking activity towards fibrin which is rendered insoluble in urea or TCA solutions. With this assay no FXIII activity was detectable with a limit of detection of 2%.

**Table 1**

| Transglutaminase levels in plasma of FXIII defective mice and normal control mice | |
|---|---|
| | Transglutaminase activity as % of standard human plasma |
| normal mice | 134% ± 10.4% |
| heterozygous FXIII defective mice | 70% ± 13.1% |
| homozygous FXIII defective mice | < 6%* |

| | |
|---|---|
| * £ 2% with a clot stability assay (H.E.Karges: Blood Coagulation Factor XIII: determination by clot stability assays. In: Bergmeyer: Methods of Enzymatic Analyses, Third Ed. Vol.V; Verlag Chemie, Weinheim, 1984; pp 400-410) | |

### - Use of the FXIII defective mouse - as a model for impaired wound healing and bleeding

FXIII defective mice were anaesthetized with hexobarbital and the dorsal area was shaved. A full thickness midline incision of the skin was made with a surgical blade along the spine. The wound was then closed with clips and the mice were allowed to wake up from anaesthesia.

Two days later the clamps were removed. At 7, 9, and 16 days after wounding the tensile strength of the healed wound was investigated for each animal.

Mice were sacrified and the dorsal skin was removed. The skin was then cut into pieces of exactly one centimeter width and 3 centimeter length with centrally located incisions. The skin piece was then introduced into a tensiometer (Hounsfield Test Equipment, Salford, Redhill, England) and the tensile strength of the incision wound was measured according to the method described by Mustoe et. a. (Science, vol. 237, 1333-1336, 1987). A force was applied across the incision by tearing with a constant speed. The force was recorded graphically and the breaking strenght was defined as the point of maximum stress before wound separation.

The breaking strength was given in Newton (N). As a control we used a mouse strain with the same genetic background without the inserted defect gene (= normal mice), which underwent the same experimental procedure.

Table 2 shows the results of the wound healing study. Compared to the control there was lower breaking strength in the FXIII defective mice when compared to normal mice. This clearly indicates an impaired wound healing.

**Table 2**

| Breaking strength of skin pieces from FXIII defective mice and normal control mice (Strain CBA) | | | |
|---|---|---|---|
| | normal mice (N) | FXIII defective mice (N) | |
| day 7 | 1.03 ± 0.09 | 0.69 ± 0.11 | |
| day 9 | 2.3 ± 0.22 | 1.46 ± 0.14 | |
| day 16 | 5.2 ± 0.03 | 3.3 ± 0 | |

The test of the bleeding time serves as a measure of the hemostatic function. For the bleeding time the tail was resected at about 2 mm apart from the tip (tail tip bleeding). The time until cessation of bleeding was monitored by blotting the blood from the wound with a filter paper. Bleeding time was given in seconds for termination of bleeding. Table 3 denotes the results of the bleeding time. FXIII defective mice had a significantly prolonged bleeding time in comparison to normal mice.

**Table 3**

| Bleeding time in FXIII defective mice and normal mice | |
|---|---|
| | Bleeding time (sec.) |
| normal mice | 170 ± 3 |
| FXIII defective mice | 274 ± 50 |

## Claims

1. A transgenic non-human mammalian animal which is heterozygous or homozygous for an at least partially defective coagulation factor XIII gene.

2. A transgenic animal according to claim 1 in which the defective gene has been inactivated by an insertion, deletion, substitution or inversion or other suitable genetic manipulation.

3. A transgenic animal according to claim 1 or 2 in which the defective coagulation factor XIII gene has been prepared by a targeted disruption of the gene for the factor XIIIa with deletion of the active site encoding the exon 7 sequence.

4. A transgenic animal according to claim 1 to 3 which is a rodent.

5. A transgenic animal according to claim 4 which is a mouse.

6. A transgenic factor XIII defective mouse characterized by the deletion of the factor XIIIa exon sequence.

7. A method of producing a transgenic animal in accordance with claims 1 to 6 the method comprising:
1. preparing a DNA construct encoding an at least partially defective coagulation factor XIII gene;
2. introducing such DNA construct into suitable carrier cells;
3. identifying cells in which the construct has integrated by homologous recombination;
4. inserting those targeted cells into an embryo;
5. placing the embryo into a mother animal, and allowing the embryo to develop to full term;
6. deriving offsprings heterozygous for the disrupted factor XIIIa gene, and
7. interbreeding heterozygous offsprings to obtain animals homozygous for the defective factor XIIIa gene.

8. A method of testing the pharmacological properties of a compound in relation to its coagulation factor XIII activity, the method comprising contacting a transgenic animal as claimed in claims 1 to 6 with said compound and monitoring its effect on disturbances of the wound healing process and bleeding disorders.

9. Use of transgenic animal as claimed in claims 1 to 6 for the investigation of orally or parenterally administered pharmaceuticals on wound healing, bleeding or other diseases associated with a deficiency in factor XIII.


### Annex to the application documents - subsequently filed sequences listing

SEQUENCE LISTING:

(1) GENERAL INFORMATION:

(i) APPLICANT:

(A) NAME: Centeon Pharma GmbH

(B) STREET: Emil-von-Behring-Strasse 76

(C) CITY: Marburg

(D) STATE: Hessen

(E) COUNTRY: Bundesrepublik Deutschland

(F) POSTAL CODE (ZIP): 35041

(G) TELEPHONE: +49(0)6421-39-2069

(H) TELEFAX: +49(0)6421-39-4558

(ii) TITLE OF INVENTION: A Transgenic Coagulation Factor XIII Defective Animal
and its Use for Testing Wound Healing and Bleeding

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 174 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(iv) ANTI-SENSE: NO
(v) FRAGMENT TYPE: internal
(vi) ORIGINAL SOURCE:
(A) ORGANISM: Mus musculus

(viii) POSITION IN GENOME:
(B) MAP POSITION: Exon 7
(C) UNITS: bp

(ix) FEATURE:
(A) NAME/KEY: exon
(B) LOCATION: 1..174

(xi) SEQUENCE DESCRIPTION: SEQ ID NO: 1:

GTGAATGCCA AGGATGATGA AGGTGTTCTT GTTGGATCAT GGGACAATGT CTATGCCTAC 60
GGCTCCCTTC CATCAGCCTG GACAGGAAGT GTTGACATTC TACTAGAATA CAGAAGCTCG 120
GAAACACCAG TCCGATATGG CCAGTGTTGG GTTTTTGCTG GTGTCTTTAA CACA 174

(2) INFORMATION FOR SEQ ID NO: 2:

(i) SEQUENCE CHARACTERISTICS:
(A) LENGTH: 174 base pairs
(B) TYPE: nucleic acid
(C) STRANDEDNESS: single
(D) TOPOLOGY: linear

(ii) MOLECULE TYPE: DNA (genomic)
(iii) HYPOTHETICAL: NO
(iv) ANTI-SENSE: NO
(vi) ORIGINAL SOURCE:
(A) ORGANISM: Rattus rattus

(viii) POSITION IN GENOME:
(B) MAp POSITION: exon 7
(C) UNITS: bp

(ix) FEATURE:
(A) NAME/KEY: exon
(B) LOCATION:1..174

(xi) SEQUENCE DESCRIPTION: SEQ ID NO: 2:

GTGAATGCCA AGGATGACGA AGGTGTTCTT GTTGGATCAT GGGACAATGT CTATGCCTAC
60
GGATCCCTTC CATCAGCCTG GACAGGAAGT GTTGACATTC TACTAGAATA CAGAAGCTCA
120
GAAACACCAG TCCGATATGG CCAGTGCTGG GTTTTTGCTG GTGTCTTTAA CACA 174

SEQUENZPROTOKOLL

<110> Centeon Pharma GmbH

<120> A Transgenic Coagulation Factor XIII Defective Animal
      and its Use for Testing Wound Healing and Bleeding

<130> C9P11EP

<140>
<141>

<160> 2

<170> PatentIn Ver. 2.1

<210> 1
<211> 174
<212> DNA
<213> Mus musculus

<400> 1
gtgaatgcca aggatgatga aggtgttctt gttggatcat gggacaatgt ctatgcctac 60
ggctcccttc catcagcctg gacaggaagt gttgacattc tactagaata cagaagctcg 120
gaaacaccag tccgatatgg ccagtgttgg gtttttgctg gtgtctttaa caca       174


<210> 2
<211> 174
<212> DNA
<213> Mus musculus

<400> 2
gtgaatgcca aggatgacga aggtgttctt gttggatcat gggacaatgt ctatgcctac 60
ggatcccttc catcagcctg gacaggaagt gttgacattc tactagaata cagaagctca 120
gaaacaccag tccgatatgg ccagtgctgg gtttttgctg gtgtctttaa caca       174